# EUROPEAN PATENT SPECIFICATION

(11) **EP 3 384 690 B1**
(45) Date of publication and mention of the grant of the patent: **10.08.2022**
(21) Application number: 16871216.4
(22) Date of filing: 05.10.2016
(51) Int. Cl.: H04L 67/12, H04W 4/70, H04B 5/00, A61B 5/00, G16H 40/67, H04W 4/80

(54) **WIRELESS NETWORK TRANSFER**
DRAHTLOSNETZWERKTRANSFER
TRANSFERT DE RÉSEAU SANS FIL

(30) Priority: 30.11.2015 US 201514954154
(43) Date of publication of application: 10.10.2018
(73) Proprietor: General Electric Company, Schenectady, NY 12345 (US)
(72) Inventor: HASAN, SM Shajedul, Niskayuna New York 12309 (US); WIK, Steven William, Niskayuna New York 12309 (US); DAVENPORT, David Michael, Niskayuna New York 12309 (US); OBI, Aghogho Atemu, Niskayuna New York 12309 (US); SORO, Stanislava, Niskayuna New York 12309 (US)
(74) Representative: Fennell, Gareth Charles
(86) International application number: PCT/US2016/055477
(87) International publication number: WO 2017/095523

(56) References cited:
- WO-A1-2014/027288
- US-A1- 2009 034 591
- US-A1- 2013 017 791
- US-A1- 2013 195 083
- US-A1- 2013 316 652
- US-A1- 2014 343 967
- US-A1- 2015 123 786
- "6 support Medical Body Area Networks (MBANS) services 7 operating in the 2360-2400 MHz band 8 Amendment: Alternative Physical Layer Extension to ; d1P802-15-4j_Draft_Standard", IEEE DRAFT; D1P802-15-4J_DRAFT_STANDARD, IEEE-SA, PISCATAWAY, NJ USA, vol. 802.15.4j, 27 March 2012 (2012-03-27) , pages 1-53, XP017765251, [retrieved on 2012-03-27]

## Description

### FIELD

The subject matter disclosed herein relates to generally to the field of data monitoring, and more specifically, to network data transfer for mobile patient monitoring.

### BACKGROUND

During the transfer of patients throughout a medical facility, medical data of a patient is monitored. The direct cable connectivity of the patient to the medical monitoring systems, however, limits a patient's mobility and restricts movement. The creation of wireless monitoring systems in hospitals has permitted greater mobility of patients, but remains restrictive because of the lack of systems, specifically network transfer systems, that permit ease of use when transferring patients from one location to another.

While wireless data transmission can offer the advantage of substantially continuous monitoring, the lack of continuity when disconnecting and reconnecting the patient from stations/hubs presents additional issues. Other devices may emit fields or signals that interfere with the ability of a wireless transmitter to accurately transmit data. Or, data networks may interfere with one another. It would be preferable to have a system that has the portability of wireless data transmission, security for data networks within a medical setting, and further has the operability that eliminates complexities of unplugging individual connections from one system and re-plugging the individual connections into another system.

A need exists to create a wireless network transfer system, secure and user friendly. The system will further allow improved patient mobility, reducing the time to transfer a wireless network among multiple monitoring devices and improving workflow when moving patients from one location to another.

US 2013/195083 is directed to a main hub, a sub hub, and a sensor node communicating in a wireless body area network (WBAN) including at least one sub hub, and a communication method thereof. A communication method of the main hub, includes assigning a beacon slot to the sub hub. The method further includes receiving, from the sub hub, a beacon signal based on the beacon slot. The method further includes verifying whether the sub hub includes data to be transmitted to the main hub based on the beacon signal. The method further includes receiving, from the sub hub, the data based on a result of the verification.

SUMMARY [0005A] The invention is defined by an independent system claim (claim 1), an independent method claim (claim 10), an independent device claim in the form of a transmitting hub (claim 12), and an independent device claim in the form of a receiving hub (claim 13). Further embodiments are specified in the dependent claims.

The above and other drawbacks or deficiencies may be overcome or alleviated by development of a system as described as follows.

The invention is focused toward enhancing a patient's mobility by providing seamless information/data transfer between medical monitoring devices such as a patient monitor, telemetry hubs/devices transfer monitor, or another mobile monitoring system. For exemplary purposes, and not limitation, patient monitors, medical monitoring devices, and mobile monitoring systems can act as the described hub presented herein. The information or data, which are transferred among the medical devices, include but are not limited to patient demographic information, wireless network, and/or pairing information. For example, a set of wireless sensors (e.g. electrocardiograph (ECG), noninvasive blood pressure (NIBP), Temperature, Oxygen Saturation (SpO2), etc.) are located on a patient's body and connected wirelessly to a patient monitor, which is not a mobile device. If a clinician needs to move the patient from a particular location to a another location, then instead of (1) disconnecting or un-pairing all the sensors from the fixed monitor and (2) connecting/pairing all of them again to a new mobile monitor, the clinician can bring the mobile monitor close to the fixed monitor to transfer the patient and associated wireless network information to the new mobile monitor automatically. This automatic wireless network and information transfer drastically improves and simplifies the workflow to move patients from one location to another one. In summary, various embodiments of the invention enable the transfer of connected on-body wireless sensor networks from one medical device to another medical device without physically detaching them from a patient's body.

### BRIEF DESCRIPTION OF THE DRAWINGS

**FIG. 1** depicts a perspective of an embodiment of the invention.
**FIG. 2** depicts a schematic to illustrate a perspective embodiment of the system.
**FIG. 3** is a schematic to illustrate one perspective embodiment of the invention.

### DETAILED DESCRIPTION

Various embodiments will be described more fully hereinafter with reference to the accompanying drawings. Such embodiments should not be construed as limiting. For example, one or more aspects can be utilized in other embodiments and even other types of systems and methodologies. Referring to the drawings in general, it will be understood that the illustrations are for the purpose of describing particular embodiments and are not intended to be limiting.

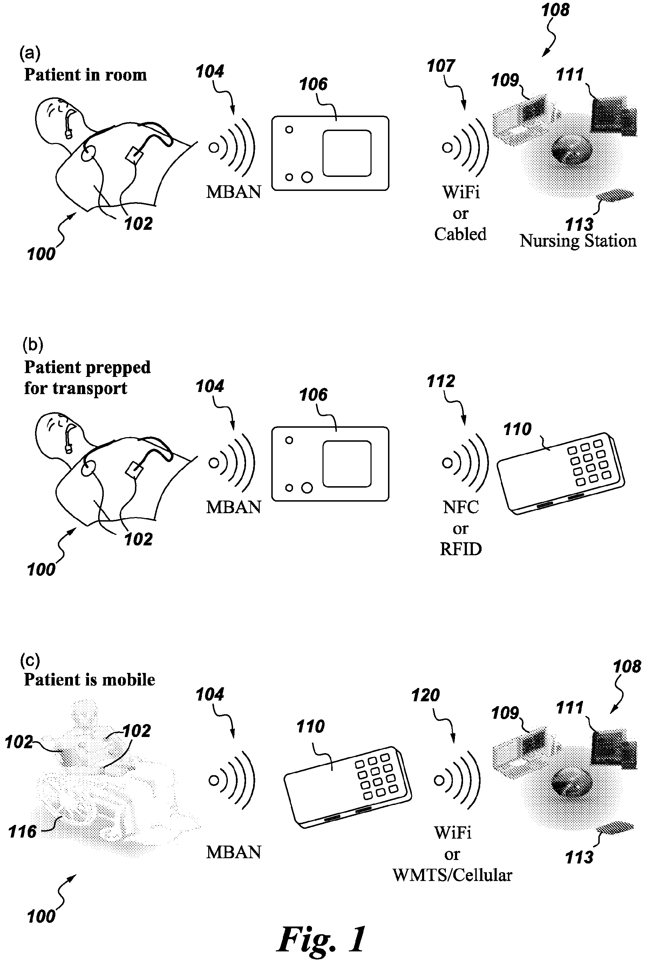

FIG. 1 depicts the system as utilized for patient monitoring in a hospital setting. At a patient 100, sensors 102 are connected. The sensors 102 are wireless and include patient monitoring information, recorded physiological parameters, diagnostic or therapeutic functions, among others. A medical body area network (MBAN) 104 interconnects communications of the sensors to a monitoring device 106, which in turn is directly connected wirelessly through WiFi 107, or by cables and wires, to a workstation 108, as shown in FIG. 1(a). Here, the monitoring device 106 is a transport monitor device with display. The workstation can include a host computer 109, server 111, or router 113 as well. The patient 100 is in one room while the workstation 108 monitors the patient 100 from a different room or station 108. The MBAN is a flexible platform for wireless networking of multiple body transmitters used for the purpose of measuring and recording physiological parameters and other patient information, or for performing diagnostic or therapeutic functions, primarily in healthcare facilities. The MBAN enhances patient safety, care and comfort, reducing the need to physically connect sensors to essential monitoring equipment via cables and wires.

Specifically, an MBAN is a low power network of sensors worn on the body controlled by a hub device that is located either on the body or in close proximity to it. The MBAN devices operate on a secondary basis in the 2360-2400 MHz band to prevent harmful interference to and must accept interference from federal and non-federal stations operating in the band. MBAN devices support wireless non-voice data communications between body-worn medical sensor (client) devices and a dedicated programmer/control (P/C) device. The sensor devices collect patient physiological information and/or deliver diagnostic and therapeutic functions. The P/C device aggregates the patient data that it receives from its associated sensors and transmits it to one or more monitoring stations via technologies that do not use the 2360-2400 MHz band (such as the hospital's local area network (LAN)). The 2360-2400 MHz frequency band is authorized by the Federal Communication Commission (FCC) for use by MBAN devices: MBAN devices in the 2360-2390 MHz band are permitted for use indoors within a health care facility, while MBAN devices in the 2390-2400 MHz band may be used anywhere. The MBAN devices in the 2360-2390 MHz band comply with a control message that notifies the P/C device(s) to limit transmissions to coordinated frequencies or to cease operation on frequencies within this band segment. An FCC-designated control point, unique to each health care facility, designates the frequencies available in the 2360-2390 MHz band at that facility. The control point uses this information to generate a control message to convey the available frequencies to the MBAN P/C device(s) via technologies that do not use the 2360-2400 MHz band (typically, the hospital's LAN). The P/C devices then use this information to enable their associated MBAN sensors on the coordinated frequencies. A P/C device will not commence operating and will automatically cease operating in the 2360-2390 MHz band if it does not receive, in accordance with the specified design protocols, a control message permitting such operation.

The MBAN may also be referred to as a wireless body area network (WBAN) or a body sensor network (BSN), as a wireless network of wearable computing devices. These devices may be adjacent to the patient, located inside the body (e.g., implants), may be mounted on a surface of the body in a fixed or movable position. The WBAN implement communications of various sorts on, near, or around the human body. Thus, the WBAN may be implemented immediate or proximate to a patient, a physician, or any personnel within or outside a medical facility. In another aspect, the WBAN can be incorporated in any home health monitoring or transport monitoring as well.

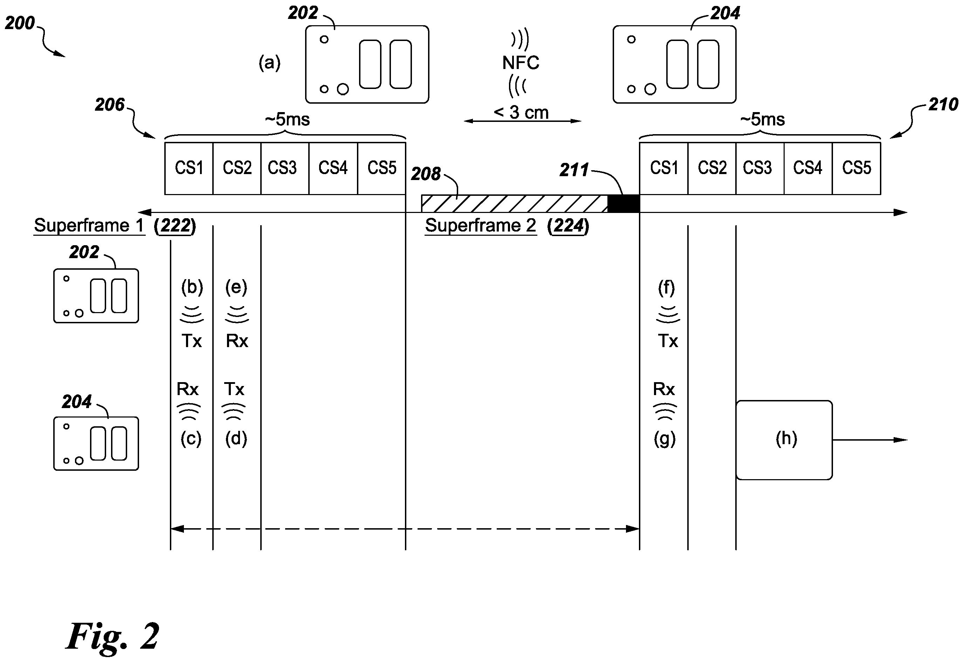

At FIG. 1(b), an ambulatory hub 110 is brought within close proximity to the ambulatory hub 110 and/or monitor 106 to pair or transfer the wireless network to the ambulatory hub 110 and the monitor 106, independently or cooperatively. The near field communications (NFC) compatibility is a contactless communication that avoids having to go through multiple steps of setting up a connection, and further avoids interruption of continuous data monitoring. The NFC technology includes sets of protocols that enable electronic devices to establish radio communication with each other by touching devices together or bringing the devices into close proximity to a distance of typically about 10 cm or less, or even closer at about 3 cm or less. When the patient 100 leaves the patient's room and becomes mobile, a nurse or doctor pairs the ambulatory hub 110 with the monitoring device 106, bringing the hub and the monitoring device into close proximity (*as shown in* *FIG. 2*). The MBAN communication network is transferred through a combination of NFC pairing and sensor rescue protocols (*See* *FIG. 2**)*, allowing the connected sensors 102 to transfer wirelessly from one hub 110 to another.

At FIG. 1(c), the patient 100 has been physically transferred to a wheelchair 116. The MBAN network has been transferred to the hub 110. The network continues communication with other remote monitoring devices and workstation 108.

Moreover, the MBAN network (104) is transferred from the monitor (106) to the ambulatory hub (110) through a combination of protocol commands executed on the NFC interface and the MBAN radio interface. The NFC interface referred to here is the interface within the NFC device between an NFC controller and the device's main application processor, defining a common level of functionality and interoperability among the components with the NFC-enabled device. As such, the interface can be utilized for various types of NFC-enabled devices, including mobile phones, computers, tablets, printers, consumer electronics, appliances, among others. The ambulatory hub 110 maintains communications with the wireless network 120 for ongoing communications with the workstation 108. The wireless network 120 includes WiFi, Wireless Medical Telemetry Service (WMTS), and/or other cellular communication networks. The WMTS includes the spectrum of frequencies used for remote monitoring of a patient's health, including devices to measure a patient's vital signs and other health parameters (e.g. pulse and respiratory rate), as well as devices that transport the data via radio (e.g. wireless cardiac monitors to monitor a patient after surgery).

In one embodiment, the hubs may be places of data convergence; in such instance, data arrives from one or more directions and is forwarded out in other directions. The ambulatory hub 110 may also include a switch to determine how and where data is forwarded from the hub, and the hub can also include a router. The networked hub has a backbone including a main circuit to which a number of outgoing lines are attached, each outgoing line providing one or more connections to a port for a device to attach to. Other networks may feed into the hub including bus networks, using a bridge to connect local area networks (LANs) and virtual local area networks (VLANs), or ring networks having a circuit arrangement in which each device is attached along the same signal path to at least two other devices. The nodes of the network are connected to a common transmission medium for linear or distributed bus network topology. In regards to a serial bus, the serial device described herein has two serial pins, the receiver, RX, and the transmitter, TX.

In one aspect, the hubs 110 connect wirelessly with the transport monitoring device 106 via NFC 112. In another aspect, any wireless communication may be utilized, including visible light communication (VLC), acoustic, RFID, infrared (IR) communication, among others. Once the hub is brought into close proximity to sensors 102 of the patient, or in proximity to the patient monitoring device 106, continuous patient monitoring is made possible. This monitor to monitor transfer also refers to any NFC communication device. The VLAN allows network administrators to partition the networks to match functional and security requirements of the devices with any changes in the network infrastructure.

The ambulatory aspect of the hub refers to a patient's mobility to walk and move around, without confining the patient to any single hospital room, floor or care area. Ambulatory care or treatment here refers to care provided to a mobile patient, moving from one room to another, to an operating room, to a diagnostic system location (e.g., Xray location), and mobility inside or outside a hospital setting. In a broad context, ambulatory is interpreted as disclosed herein to also refer to movement of a patient in "outpatient" care, outside the hospital or medical facility.

FIG. 2 illustrates an embodiment of an ambulatory hub transfer system 200. At FIG. 2(a), a first ambulatory hub 202 enabled with a 7-byte NFC tag unique identifier (UID) comes into close proximity with a second ambulatory hub 204 which includes an NFC reader. The second ambulatory hub 204 detects that the NFC tag UID of the first ambulatory hub and has been read through a NFC reader in 204.

As depicted, a superframe 222 comprises time division medium access control (MAC) protocols for managing data exchange of the sensors 102 over the MBAN radio network (104). Multiple superframes can be included in the system for durations of time as desired by the user. The superframe 1 (222) includes an overall time division protocol of about 100 msec or less. Time division protocols less than 100 msec enable efficient and controlled transfer of data, as utilized when quickly mobilizing a patient. Every timeslot in the superframe 222 has a different assigned functionality; for exemplary purposes, and not limitation, five slots CS1-CS5 are dedicated to MAC-level management and control, and referred to as control slots 206. Control slots 206 are timeslots in the superframe 222 dedicated to "control-like" functionalities in the wireless protocol. The slots, or control slots 206 referred to here are MAC protocols for managing data exchange, also referred to as management slots. Control slots 206 (i.e., CS1, CS2, CS3, CS4, CS5...etc.) control the transfer of MBAN data between the first ambulatory hub 202 and second hub 204. The plurality of control slots 206 are organized into superframe 1 (222), superframe 2 (224), and so on.

Examples of control-like functionalities are association and disassociation (e.g., CS1 and CS2), and rescues (i.e., reconnecting lost connections) and/or advertisements (ADV) (i.e., where a hub sends a transmission so that any nearby sensors can detect the presence of the hub), as in CS3. These control-like activities may be implemented and modified as desired, as dependent on a user's experience. In addition, any wireless protocol may be implemented, including WiFi and Bluetooth, among others that perform control-like activities in order to start, maintain, and continuously monitor the wireless link between two devices. Various activities in the control slots may serve purposes as described here, or may further be modified as designated for particular healthcare monitoring functions, diagnosis, and operability of user systems internal or external to a medical setting.

In an embodiment of a protocol using the system 200, each of the control slots, CS1, CS2, CS3, CS4, and CS5 is about 1 msec long; and as part of a superframe of about 100 msec duration, the control slots 206 in combination utilize about 5 msec of time. The control slots are scheduled towards the end of the superframe, though different arrangements may be utilized depending on timing connection, and use of monitoring functionalities. As such, the control slots may manage the transfer of the network at the beginning, end, or anytime therebetween within the time divided wireless protocol.

A variety of slot types within the superframe are arranged and combined in connection with patient data, and are different from the control slots. The combination of slots 208, including beacon slots and sensor data slots, are used for sending and receiving actual sensor data from the connected patient sensors 102. For example, an ECG sensor may be assigned sensor data slots 1-15 earlier in the superframe, and sends its data to the hub in those timeslots. Before the ECG sensor starts transmitting in those slots, the sensor initiates association (or pairing) which is done in the control slots. It is through this process that the ECG sensor "connects" wirelessly to the hub 202 and is assigned those specified slots for transmitting the sensor data.

In FIG. 2, superframe 2 (224) comprises a beacon and sensor data slots 208, with control slots 210 at the end of the superframe. Blank slots 211 may be left open for other designated activity, as may be implemented in the time division protocols at any time. In one aspect, the beacon and sensor data slots 208 are not utilized for the connect protocol. In another aspect, the system 200 may be modified to further include the beacon and sensor data slots in various arrangements within the time divided protocol to further facilitate process and workflow. As demonstrated, in one aspect, the last few milliseconds of time in the superframe 224 comprise blank slots 211 not used for any wireless activities. Aspects of the invention may be modified and revised as desired, such modifications to include variation in time slots, or rather time division protocol. As illustrated, drawings are depicted to clearly illustrate features of the invention, and not necessarily drawn to scale.

During network transfer, the hub 202 is brought into close proximity to the hub 204. The first ambulatory hub 202 enables the transmit and receive mode (TX/RX) on CS 1 and CS2 for about three (3) seconds or less. Any duration of TX/RX may be modified as desired for a specified system. During CS1, the first ambulatory hub 202 TX association is made with the second ambulatory hub 204 RX. During CS2, node TX association is made with the first ambulatory hub 202 RX. The node, as part of data terminal equipment (DTE) is a connection point, redistribution point, or communication endpoint. The DTE may be a digital mobile device, host computer, router, workstation, or server, among other communication endpoints. In addition, a hub, bridge, modem, or switches are referred to herein as data communication equipment (DCE), as active electronic devices attached to the network.

Specifically, at FIG. 2(b), the ambulatory hub 202 transmits (TX) on CS1 includes an operation code (opcode) to be performed which identifies a packet type of data as "NFC pairing". A payload of data from the hub 202 TX includes a UID of the read NFC tag on the second ambulatory hub 204; the ambulatory hub 204 with this UID, at FIG. 2(c), is then allowed to receive data in the same slot CS1. FIG. 2(d) depicts that the ambulatory hub 204, having received the "NFC pairing" packet in CS1, copies the connected sensor and/or node UID from the "NFC pairing" packet to its rescue node or sensor list. The rescue node and/or sensor list provide reconnection or recovery of lost signals and/or connections. The second ambulatory hub 204 then transmits back to the first hub 202 with the opcode: "op_control_hub_to_hub_swap_req,", indicating that it would like to swap its network with the original first hub.

At FIG. 2(e), in CS2, the first hub 202 receives (RX) the transmission from the second hub 204, recognizing that the second ambulatory hub 204 has received the first ambulatory hub 202 packet and detecting that it is a hub-to-hub swap request. The first ambulatory hub 202 then internally disassociates its attached nodes and/or sensor units. This internal disassociation involves hub 202 notifying its attached sensor or node units that they have been removed from the hub's connection.

At FIG. 2(f), in CS1 of the next superframe 2 (224), the first hub 202 transmits an acknowledgement code (ACK) to the swap request received in FIG. 2(e). The ACK is a transmission control character to indicate that the previously transmitted message was received, uncorrupted and/or without errors. The receiver in this case, hub 202, sends the ACK code to the sender (hub 204) to indicate that the previous transmission has been accepted. FIG. 2(g) identifies where the second ambulatory hub 204 receives the ACK packet in CS1 of superframe 2, and restarts the superframe timer; this enables rescues or automatic sensor re-association of the previously associated nodes and/or sensors of the prior hub (202) for up to about 90 seconds.

In this process of rescue and re-association, the second hub 204 begins sending rescue codes in CS3 of its superframe, along with the sensor or node unit UIDs of the sensor or node unit(s) previously connected to the first hub 202. This process is done in order to rescue and connect the sensor or node units to the network of the second hub 204; thus, this completes the transfer of the network of the first hub 202 to the network of the second hub 204. The first ambulatory hub 202 TX/RX at FIG. 2(h) ceases upon successful swap or after about a three (3) second timeout, and has no sensor units attached.

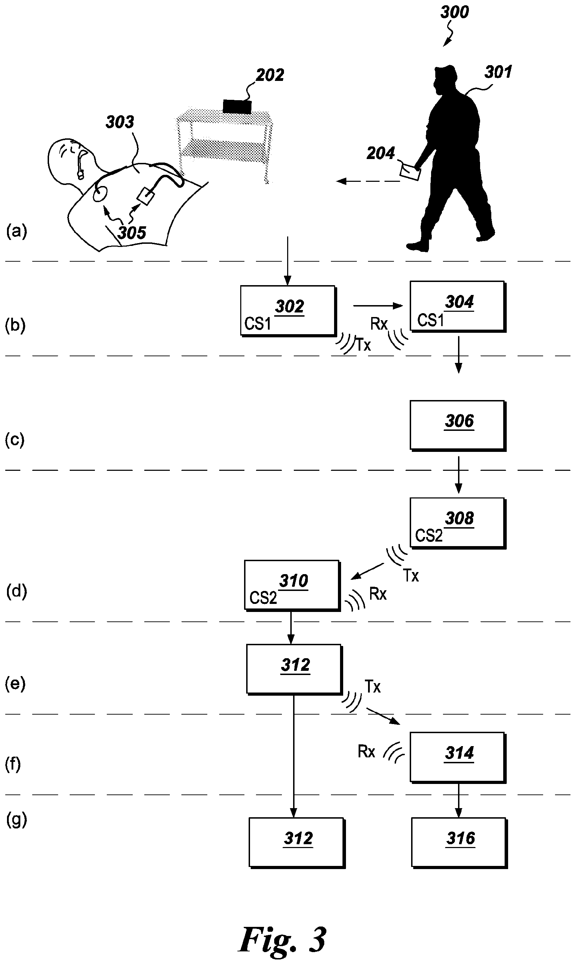

FIG. 3 is a schematic of the system 300 such that a clinician 301 brings ambulatory hub 204 into close proximity with hub 202 to which a patient 303 has sensors 305 wirelessly connected to the hub 202. Hub 202 in turn prepares to transfer its network to hub 204 to monitor the patient's sensors during transport of the patient from the room, and continuously monitor the patient while the patient is mobile.

Once the hub 204 is brought within about 10 cm of the hub 202, (a) the NFC tag UID of hub 202 is in communication with hub 204; hub 204 detects the NFC tag UID of hub 202; (b) at CS1, hub 202 transmits a packet (302) with an opcode identifying the packet as "NFC pairing." In the same slot CS1, hub 204 receives (304) the "NFC pairing" packet from hub 202. At FIG. 3(c), hub 204 copies (306) the list of hub 202's connected node and/or sensor UIDs from the received "NFC pairing" packet to its rescue node/sensor lists, permitting reconnect and recovery operations; (d) a swap network request is sent out (308) at CS2 in response to hub 202's initial "NFC pairing" packet of hub 202. Hub 202 receives (310) the swap network request in the same slot CS2 and internally dissociates all of its node and/or sensor units from its network; (e) hub 202 transmits (312) an acknowledgement (ACK) of the swap request; (f) hub 204 receives (314) the ACK request; (g) hub 202 ceases (312) communications with hub 204 and hub 204 initiates (316) the process to connect to the sensors and/or node units previously associated with hub 202.

Information privacy, especially in healthcare, is also maintained during the exchange of networks. Communications at about 2.4 GHz on control slots 1-5 occur on a predefined frequency using a protected key(s). Various security measures are accounted for in the exchange allowing a user-friendly, one touch or no touch interface while securely and effectively transferring patient information on data networks to and from ambulatory hubs. The information remains retrievable from workstation, nursing stations, and mobile devices within a specified network.

One aspect of the invention allows seamless data transfer and wireless network handover among medical devices, stations, and monitors. When a clinician wants to transfer the wireless network among the different patient monitors, the monitors can be set in transfer mode by simplistic pressing of a button. When a monitor is in transfer mode, then the handover process can happen using: (1) A first method that employs NFC technologies or passive/active RFID. Through this method when a patient monitor touches or comes close to another patient monitor, the wireless network and other data transfer happens automatically. (2) Point to point wireless communication techniques can be used as a second method to transfer the network handover between devices. Wireless protocols, which can be used for this point to point data transfer, include but are not limited to WiFi, Bluetooth, Zigbee or other communication protocol. (3) A third technique utilizes a network cloud. Monitors or medical devices can be connected to a cloud through wired or wireless communication. When transfer mode is enabled, then one monitor hands over the connected devices to another monitor through network cloud infrastructure. (4) Another method includes using optical communications (e.g., visible light or infrared) or acoustic to provide communication between medical devices. Communication in visible and infrared range is suitable here since it overcomes the interference problems associated with radio-frequency (RF) communication technology; the visible and infrared ranges enable communications between devices within a direct line of sight (i.e., located in the same room). Similar remarks can be made about the use of high and low frequency acoustic signals where interference does not present issues.

The system improves patient mobility, reducing the time to transfer a wireless network among monitoring devices. The workflow is improved as the patient can be monitored during transfer from one location to another.

Embodiments of the invention may also be developed and validated by physically connecting cables between the monitors for handing over network information, or having a combination of cable and cableless monitoring networks.

In addition, the hubs may take the shape of any structure, any size and configuration. In one aspect, the hub includes a housing. In another aspect, the hub uses printed technologies with flexible substrates, textiles and adhesives, and therefore no housing.

Furthermore, any embodiment of the time divided protocol could use modifiable repeating and/or noncontinuous or nonrepeating frame structures where the superframe is referred to herein. The repetitive superframes described and depicted in the embodiments above, are for exemplary purposes, and not limitation. In addition, the choice of the time duration is system-dependent, as based on usability, protocol management, and as desired for efficacy and efficiency.

Advantageously, the network transfer between the hubs is initiated automatically when hubs are brought in close proximity to one another. In another aspect, embodiments of the network swap may initiate the transfer by way of user interaction, such as push button, voice command, optical or other signaling. Various aspects may thus be modified to incorporate another initiation process for the network swap, as desired by the user.

The invention is defined by the appended independent claims.

## Claims

1. A system for managing transfer of data in a medical body area network, MBAN, (104) of one or more sensors (102) to wirelessly mobilize a patient, the one or more sensors (102) each comprising a sensor node having a unique identifier and a corresponding network address, the system comprising:
a plurality of hubs, including at least a first hub (202) and at least a second hub (204), each hub having a dual communication interface including an MBAN interface and a short range interface,
wherein the first hub (202) is configured to connect to the one or more sensors of the MBAN (104) through its MBAN interface,
wherein each hub comprises a wireless transceiver and a processor configured to manage a time division protocol comprising:
one or more data timeslots to send or receive data from the one or more sensors (102), and
one or more control timeslots designated to manage transfer of the MBAN within the time division protocol from the first hub (202) to the second hub (204), the control timeslots including a transmit mode and a receive mode,
wherein the first hub (202) is configured to:
be associated with the one or more sensors,
enable, when in proximity to the second hub (204), the transmit and receive modes, and transmit an NFC pairing packet of data within the one or more control timeslots, including the unique identifier and corresponding network address of the one or more sensors in the MBAN (104), from the first hub (202) to the short range interface of the second hub (204), and receive a swap network request from the second hub (204),
wherein the second hub (204) is configured to:
receive the NFC pairing packet of data within the one or more control timeslots, and
send (308) the swap network request packet to the first hub (202) in response to the NFC pairing packet received from the first hub (202).

2. The system of claim 1, wherein the time division protocol includes identification of the unique identifiers and corresponding network addresses of the sensor nodes, and then transfers the unique identifiers and the corresponding network addresses from the first hub (202) to the second hub (204) in combination.

3. The system of claim 1, further comprising one or more monitoring devices configured to interconnect with the MBAN interface to monitor the data, optionally wherein the MBAN (104) is configured to communicate with the one or more sensors and to relay data to the one or more monitoring devices in real-time.

4. The system of claim 1, wherein the time division protocol allows the hubs to manage the MBAN interface and the short range interface through a series of the one or more control slots.

5. The system of claim 1, wherein the short range interface is a near field communication, NFC, interface.

6. The system of claim 1, wherein the short range interface comprises magnetic fields, electric fields, optics, acoustic, ultrasound, and mechanical vibrations.

7. The system of claim 1, wherein the time division protocol is organized into a plurality of frames, each frame comprising the one or more control timeslots.

8. The system of claim 1, wherein the one or more control timeslots comprise functionalities of detection, association, dissociation, reconnect, rescue, network swapping, and advertisement.

9. The system of claim 1, wherein the one or more sensors are adjacent to a patient, configured to attach to a surface of a body of the patient, or located inside the body of the patient.

10. A method of managing transfer of data of a medical body area network, MBAN, (104) of one or more sensors (102) to wirelessly mobilize a patient comprising the steps of:
providing the system of claim 1;
bringing the first hub (202) in proximity to the second hub (204) to enable the transmit and receive modes;
transmitting (302) within the one or more control timeslots an NFC pairing packet of data, including the identifier and address of the one or more sensors in the MBAN (104), from the first hub (202) to the short range interface of the second hub (204) and receive a swap network request from the second hub (204).

11. The method of claim 10, wherein the one or more control timeslots are distributed within the time division protocol.

12. A hub (202) for a medical body area network, MBAN, the MBAN comprising one or more sensors to wirelessly mobilize a patient, the one or more sensors (102) each comprising a sensor node having a unique identifier and a corresponding network address, the hub (202) having a dual communication interface including an MBAN interface and a short range interface,
the hub (202) comprising a wireless transceiver and a processor configured to
manage a time division protocol comprising:
one or more data timeslots, and
one or more control timeslots designated to manage transfer of the MBAN within the time division protocol from the hub (202) to a second hub (204), the control timeslots including a transmit mode and a receive mode,
wherein the hub (202) is configured to:
associate with the one or more sensors,
enable, when brought in proximity to the second hub (204), the transmit and receive modes,
transmit an NFC pairing packet of data within the one or more control timeslots, including the unique identifier and corresponding network address of the one or more sensors in the MBAN, from the hub (202) to the short range interface of the second hub (204), and receive a swap network request from the second hub (204).

13. A hub (204) for a medical body area network, MBAN, the MBAN comprising one or more sensors to wirelessly mobilize a patient, the one or more sensors (102) each comprising a sensor node having a unique identifier and a corresponding network address, the hub (204) having a dual communication interface including an MBAN interface and a short range interface,
the hub (204) comprising a wireless transceiver and a processor configured to
manage a time division protocol comprising:
one or more data timeslots, and
one or more control timeslots designated to manage transfer of the MBAN within the time division protocol from another hub (202) to the hub (204), the control timeslots including a transmit mode and a receive mode,
wherein the hub (204) is configured to:
receive an NFC pairing packet of data within the one or more control timeslots, including the unique identifier and corresponding network address of the one or more sensors in the MBAN (104), from the other hub (202) to the short range interface of the hub (204), wherein the one or more sensors were previously associated with the other hub (202), and send (308) a swap network request packet to the first hub (202) in response to receiving the NFC pairing packet.

## Patentansprüche

1. System zum Verwalten der Datenübertragung in einem medizinischen Körperbereichsnetz, MBAN, (104) aus einem oder mehreren Sensoren (102), um einen Patienten drahtlos zu mobilisieren, wobei der eine oder die mehreren Sensoren (102) jeweils einen Sensorknoten mit einer eindeutigen Kennung und einer entsprechenden Netzadresse aufweisen, wobei das System Folgendes umfasst:
mehrere Hubs einschließlich mindestens eines ersten Hubs (202) und mindestens eines zweiten Hubs (204), wobei jeder Hub eine duale Kommunikationsschnittstelle aufweist, die eine MBAN-Schnittstelle und eine Kurzstreckenschnittstelle umfasst,
wobei der erste Hub (202) dazu ausgelegt ist, sich über seine MBAN-Schnittstelle mit dem einen oder den mehreren Sensoren des MBAN (104) zu verbinden,
wobei jeder Hub einen drahtlosen Sendeempfänger und einen Prozessor aufweist, die dazu ausgelegt sind, ein Zeitaufteilungsprotokoll zu verwalten, das umfasst:
einen oder mehr Datenzeitschlitze zum Senden oder Empfangen von Daten aus dem einen oder den mehreren Sensoren (102) und
einen oder mehrere Steuerzeitschlitze, die dazu bestimmt sind, die Übertragung des MBAN innerhalb des Zeitaufteilungsprotokolls von dem ersten Hub (202) zu dem zweiten Hub (204) zu verwalten, wobei die Steuerzeitschlitze einen Sendemodus und einen Empfangsmodus umfassen,
wobei der erste Hub (202) zu Folgendem ausgelegt ist:
dem einen oder den mehreren Sensoren zugeordnet zu sein,
den Sende- und den Empfangsmodus zu aktivieren, wenn er sich in der Nähe des zweiten Hubs (204) befindet, und
ein NFC-Paarungsdatenpaket von Daten innerhalb des einen oder der mehreren Steuerzeitschlitze, das die eindeutige Kennung und die entsprechende Netzadresse des einen oder der mehreren Sensoren in dem MBAN (104) umfasst, von dem ersten Hub (202) zu der Kurzstreckenschnittstelle des zweiten Hub (204) zu senden und eine Netzaustauschanforderung von dem zweiten Hub (204) zu empfangen,
wobei der zweite Hub (204) zu Folgendem ausgelegt ist:
das NFC-Paarungsdatenpaket innerhalb des einen oder der mehreren Steuerzeitschlitze zu empfangen und das Netzaustauschanforderungspaket als Antwort auf das NFC-Paarungspaket, das von dem ersten Hub (202) empfangen wird, an den ersten Hub (202) zu senden (308).

2. System nach Anspruch 1, wobei das Zeitaufteilungsprotokoll eine Identifizierung der eindeutigen Kennungen und der entsprechenden Netzadressen der Sensorknoten umfasst und dann die eindeutigen Kennungen und die entsprechenden Netzadressen in Kombination von dem ersten Hub (202) zu dem zweite Hub (204) überträgt.

3. System nach Anspruch 1, das ferner eine oder mehrere Überwachungsvorrichtungen aufweist, die dazu ausgelegt sind, mit der MBAN-Schnittstelle verbunden zu werden, um die Daten zu überwachen, wobei das MBAN (104) optional dazu ausgelegt ist, mit dem einen oder den mehreren Sensoren zu kommunizieren und Daten an die eine oder mehreren Überwachungsvorrichtungen in Echtzeit weiterzuleiten.

4. System nach Anspruch 1, wobei es das Zeitaufteilungsprotokoll den Hubs ermöglicht, die MBAN-Schnittstelle und die Kurzstreckenschnittstelle durch eine Folge des einen oder der mehreren Steuerschlitze zu verwalten.

5. System nach Anspruch 1, wobei die Kurzstreckenschnittstelle eine Nahfeldkommunikations-Schnittstelle, NFC-Schnittstelle, ist.

6. System nach Anspruch 1, wobei die Kurzstreckenschnittstelle magnetische Felder, elektrische Felder, optische Schwingungen, akustische Schwingungen, Ultraschallschwingungen und mechanische Schwingungen umfasst.

7. System nach Anspruch 1, wobei das Zeitaufteilungsprotokoll in mehreren Rahmen organisiert ist, wobei jeder Rahmen den einen oder die mehreren Steuerzeitschlitze umfasst.

8. System nach Anspruch 1, wobei der eine oder die mehreren Steuerzeitschlitze Funktionalitäten von Detektion, Zuordnung, Loslösung, Wiederverbindung, Wiederherstellung, Netzaustausch und Ankündigung umfassen.

9. System nach Anspruch 1, wobei der eine oder die mehreren Sensoren zu einem Patienten benachbart sind, dazu ausgelegt sind, an einer Oberfläche eines Körpers des Patienten angebracht zu werden, oder innerhalb des Körpers des Patienten angeordnet sind.

10. Verfahren zum Verwalten einer Übertragung von Daten eines medizinischen Körperbereichsnetzes, MBAN, (104) aus einem oder mehreren Sensoren (102), um einen Patienten drahtlos zu mobilisieren, das die folgenden Schritte umfasst:
Bereitstellen des Systems nach Anspruch 1;
Bringen des ersten Hubs (202) in die Nähe des zweiten Hubs (204), um den Sende- und den Empfangsmodus zu aktivieren,
Senden (302) eines NFC-Paarungsdatenpakets, das die Kennung und Adresse des einen oder der mehreren Sensoren in dem MBAN (104) umfasst, innerhalb des einen oder der mehreren Steuerzeitschlitze von dem ersten Hub (202) zu der Kurzstreckenschnittstelle des zweiten Hub (204) und Empfangen einer Netzaustauschanforderung aus dem zweiten Hub (204).

11. Verfahren nach Anspruch 10, wobei der eine oder die mehreren Steuerzeitschlitze innerhalb des Zeitaufteilungsprotokolls verteilt sind.

12. Hub (202) für ein medizinisches Körperbereichsnetz, MBAN, wobei das MBAN einen oder mehrere Sensoren umfasst, um einen Patienten drahtlos zu mobilisieren, wobei der eine oder die mehreren Sensoren (102) jeweils einen Sensorknoten mit einer eindeutigen Kennung und einer entsprechenden Netzadresse umfassen, wobei der Hub (202) eine duale Kommunikationsschnittstelle einschließlich einer MBAN-Schnittstelle und einer Kurzstreckenschnittstelle aufweist,
wobei der Hub (202) einen drahtlosen Sendeempfänger und einen Prozessor aufweist, die dazu ausgelegt sind, ein Zeitaufteilungsprotokoll zu verwalten, das umfasst:
einen oder mehrere Datenzeitschlitze und
einen oder mehrere Steuerzeitschlitze, die dazu bestimmt sind, die Übertragung des MBAN innerhalb des Zeitaufteilungsprotokolls von dem ersten Hub (202) zu dem zweiten Hub (204) zu verwalten, wobei die Steuerzeitschlitze einen Sendemodus und einen Empfangsmodus umfassen,
wobei der Hub (202) zu Folgendem ausgelegt ist:
dem einen oder den mehreren Sensoren zugeordnet zu sein,
den Sende- und den Empfangsmodus zu aktivieren, wenn er in die Nähe des zweiten Hubs (204) gebracht wird, und
ein NFC-Paarungsdatenpaket von Daten innerhalb des einen oder der mehreren Steuerzeitschlitze, das die eindeutige Kennung und die entsprechende Netzadresse des einen oder der mehreren Sensoren in dem MBAN umfasst, von dem ersten Hub (202) zu der Kurzstreckenschnittstelle des zweiten Hub (204) zu senden und eine Netzaustauschanforderung von dem zweiten Hub (204) zu empfangen.

13. Hub (204) für ein medizinisches Körperbereichsnetz, MBAN, wobei das MBAN einen oder mehrere Sensoren umfasst, um einen Patienten drahtlos zu mobilisieren, wobei der eine oder die mehreren Sensoren (102) jeweils einen Sensorknoten mit einer eindeutigen Kennung und einer entsprechenden Netzadresse umfassen, wobei der Hub (204) eine duale Kommunikationsschnittstelle einschließlich einer MBAN-Schnittstelle und einer Kurzstreckenschnittstelle aufweist,
wobei der Hub (204) einen drahtlosen Sendeempfänger und einen Prozessor aufweist, die dazu ausgelegt sind, ein Zeitaufteilungsprotokoll zu verwalten, das umfasst:
einen oder mehrere Datenzeitschlitze, und
einen oder mehrere Steuerzeitschlitze, die dazu bestimmt sind, die Übertragung der MBAN innerhalb des Zeitaufteilungsprotokolls von einem anderen Hub (202) zu dem Hub (204) zu verwalten, wobei die Steuerzeitschlitze einen Sendemodus und einen Empfangsmodus umfassen,
wobei der Hub (204) zu Folgendem ausgelegt ist:
ein NFC-Paarungsdatenpaket von Daten innerhalb des einen oder der mehreren Steuerzeitschlitze, das die eindeutige Kennung und die entsprechende Netzadresse des einen oder der mehreren Sensoren in dem MBAN (104) umfasst, von dem ersten Hub (202) zu der Kurzstreckenschnittstelle des zweiten Hub (204) zu senden, wobei der eine oder die mehreren Sensoren zuvor dem anderen Hub (202) zugeordnet waren, und das Netzaustauschanforderungspaket als Antwort auf das Empfangen des NFC-Paarungspakets an den ersten Hub (202) zu senden (308) .

## Revendications

1. Système de gestion de transfert de données dans un réseau corporel médical, MBAN, (104) d'un ou plusieurs capteurs (102) pour mobiliser un patient par une liaison sans fil, le ou les capteurs (102) comprenant chacun un nœud de capteur ayant un identifiant unique et une adresse réseau correspondante, le système comprenant :
une pluralité de concentrateurs, incluant au moins un premier concentrateur (202) et au moins un deuxième concentrateur (204), chaque concentrateur ayant une double interface de communication incluant une interface MBAN et une interface courte-portée,
le premier concentrateur (202) étant configuré pour se connecter aux un ou plusieurs capteurs du MBAN (104) via son interface MBAN,
chaque concentrateur comprenant un émetteur-récepteur sans fil et un processeur configuré pour gérer un protocole de répartition temporelle comprenant :
un ou plusieurs créneaux temporels de données pour envoyer ou recevoir des données en provenance du ou des capteurs (102), et
un ou plusieurs créneaux temporels de commande conçus pour gérer un transfert du MBAN dans le protocole de répartition temporelle du premier concentrateur (202) vers le deuxième concentrateur (204), les créneaux temporels de commande incluant un mode transmission et un mode réception,
le premier concentrateur (202) étant configuré pour :
être associé aux un ou plusieurs capteurs, permettre, lorsqu'il se trouve à proximité du deuxième concentrateur (204), les modes transmission et réception, et transmettre un paquet de données d'appairage NFC dans le ou les créneaux temporels de control, incluant l'identifiant unique et une adresse réseau correspondante du ou des capteurs dans le MBAN (104), du premier concentrateur (202) vers l'interface courte-portée du deuxième concentrateur (204), et recevoir une demande d'échange de réseau en provenance du deuxième concentrateur (204),
le deuxième concentrateur (204) étant configuré pour :
recevoir le paquet de données d'appairage NFC dans le ou des créneaux temporels de commande, et
envoyer (308) le paquet de demande de réseau d'échange au premier concentrateur (202) en réponse au paquet d'appairage NFC reçu du premier concentrateur (202) .

2. Système selon la revendication 1, dans lequel le protocole de répartition temporelle comprend l'identification des identifiants uniques et des adresses réseau correspondantes des nœuds de capteur, puis transfère les identifiants uniques et les adresses réseau correspondantes du premier concentrateur (202) au deuxième concentrateur (204) en combinaison.

3. Système selon la revendication 1, comprenant en outre un ou plusieurs dispositifs de surveillance configurés pour s'interconnecter avec l'interface MBAN pour surveiller les données, le MBAN (104) étant optionnellement configuré pour communiquer avec le ou les capteurs et pour relayer des données en temps réel vers le ou les dispositifs de surveillance.

4. Système selon la revendication 1, dans lequel le protocole de division temporelle permet aux concentrateurs de gérer l'interface MBAN et l'interface courte-portée à travers une série des un ou plusieurs créneaux temporels.

5. Système selon la revendication 1, dans lequel l'interface courte-portée est une interface de communication en champs proche, NFC.

6. Système selon la revendication 1, dans lequel l'interface courte-portée comprend des champs magnétiques, des champs électriques, de l'optique, de l'acoustique, des ultrasons et des vibrations mécaniques.

7. Système selon la revendication 1, dans lequel le protocole de répartition temporelle est organisé en une pluralité de trames, chaque trame comprenant le ou les créneaux temporels de commande.

8. Système selon la revendication 1, dans lequel le ou les créneaux temporels de commande comprennent des fonctionnalités de détection, association, dissociation, reconnexion, secours, échange de réseau et publicité.

9. Système selon la revendication 1, dans lequel le ou les capteurs sont adjacents à un patient, conçus pour être attachés à une surface corporelle du patient, ou situés à l'intérieur du corps du patient.

10. Procédé de gestion de transfert de données d'un réseau corporel médical, MBAN, (104) d'un ou plusieurs capteurs (102) pour mobiliser un patient par une liaison sans fil, comprenant les étapes de :
mise à disposition du système selon la revendication 1 ;
rapprochement du premier concentrateur (202) à proximité du deuxième concentrateur (204) pour permettre les modes transmission et réception ;
transmission (302) dans le ou les créneaux temporels de commande d'un paquet de données d'appairage NFC, incluant l'identifiant et l'adresse du ou des capteurs du MBAN (104), du premier concentrateur (202) vers l'interface courte-portée du deuxième concentrateur (204), et réception d'une demande d'échange de réseau en provenance du deuxième concentrateur (204).

11. Procédé selon la revendication 10, dans lequel le ou les créneaux temporels de commande sont distribués dans le protocole de répartition temporelle.

12. Concentrateur (202) pour un réseau corporel médical, MBAN, le MBAN comprenant un ou plusieurs capteurs pour mobiliser un patient par une liaison sans fil, le ou les capteurs (102) comprenant chacun un nœud de capteur ayant un identifiant unique et une adresse réseau correspondante, le concentrateur (202) ayant une double interface de communication incluant une interface MBAN et une interface courte-portée,
le concentrateur (202) comprenant un émetteur-récepteur sans fil et un processeur configuré pour gérer un protocole de répartition temporelle comprenant :
un ou plusieurs créneaux temporels de données, et
un ou plusieurs créneaux temporels de commande conçus pour gérer un transfert du MBAN dans le protocole de répartition temporelle du concentrateur (202) vers le deuxième concentrateur (204), les créneaux temporels de commande incluant un mode transmission et un mode réception,
le concentrateur (202) étant configuré pour :
s'associer aux un ou plusieurs capteurs, permettre, lorsqu'il est amené à proximité du deuxième concentrateur (204), les modes transmission et réception,
transmettre un paquet de données d'appairage NFC dans le ou les créneaux temporels de control, incluant l'identifiant unique et une adresse réseau correspondante du ou des capteurs dans le MBAN, du concentrateur (202) vers l'interface courte-portée du deuxième concentrateur (204), et
recevoir une demande d'échange de réseau en provenance du deuxième concentrateur (204).

13. Concentrateur (204) pour un réseau corporel médical, MBAN, le MBAN comprenant un ou plusieurs capteurs pour mobiliser un patient par une liaison sans fil, le ou les capteurs (102) comprenant chacun un nœud de capteur ayant un identifiant unique et une adresse réseau correspondante, le concentrateur (204) ayant une double interface de communication incluant une interface MBAN et une interface courte-portée,
le concentrateur (204) comprenant un émetteur-récepteur sans fil et un processeur configuré pour gérer un protocole de répartition temporelle comprenant :
un ou plusieurs créneaux temporels de données, et
un ou plusieurs créneaux temporels de commande conçus pour gérer un transfert du MBAN dans le protocole de répartition temporelle d'un autre concentrateur (202) vers le concentrateur (204), les créneaux temporels de commande incluant un mode transmission et un mode réception,
le concentrateur (204) étant configuré pour :
recevoir un paquet de données d'appairage NFC dans le ou des créneaux temporels de commande, incluant l'identifiant unique et l'adresse réseau correspondante du ou des capteurs dans le MBAN (104), de l'autre concentrateur (202) vers l'interface courte-portée du concentrateur (204), le ou les capteurs ayant été précédemment associés à l'autre concentrateur (202), et envoyer (308) un paquet de demande d'échange de réseau au premier concentrateur (202) en réponse à la réception du paquet d'appairage NFC.
